# EUROPEAN PATENT SPECIFICATION

(11) **EP 4 060 347 B1**
(45) Date of publication and mention of the grant of the patent: **15.07.2026**
(21) Application number: 21162683.3
(22) Date of filing: 15.03.2021
(51) Int. Cl.: G01N 33/68

(54) **METHOD FOR SCREENING A SUBJECT FOR THE RISK OF CHRONIC KIDNEY DISEASE AND COMPUTER-IMPLEMENTED METHOD**
VERFAHREN ZUM SCREENEN EINER PERSON AUF DAS RISIKO EINER CHRONISCHEN NIERENERKRANKUNG UND COMPUTERIMPLEMENTIERTES VERFAHREN
PROCÉDÉ DE CRIBLAGE D'UN SUJET POUR ÉVALUER LE RISQUE D'UNE MALADIE RÉNALE CHRONIQUE ET PROCÉDÉ MIS EN UVRE PAR ORDINATEUR

(43) Date of publication of application: 21.09.2022
(73) Proprietor: F. Hoffmann-La Roche AG, 4070 Basel (CH); Roche Diabetes Care GmbH, 68305 Mannheim (DE)
(72) Inventor: RINGEMANN, Christian, 68305 Mannheim (DE); HUSCHTO, Tony, 68305 Mannheim (DE); KÖNIG, Helena, 68305 Mannheim (DE)
(74) Representative: Bittner, Thomas L.

(56) References cited:
- WO-A1-2019/180232
- LIU BI-CHENG ET AL: "Investigation of the prevalence of CKD in 13,383 Chinese hospitalized adult patients", CLINICA CHIMICA ACTA, vol. 387, no. 1, 2 October 2007 (2007-10-02), pages 128 - 132, XP029159189, ISSN: 0009-8981, DOI: 10.1016/J.CCA.2007.09.020
- LU ET AL: "High prevalence of chronic kidney disease in population-based patients diagnosed with type 2 diabetes in downtown Shanghai", JOURNAL OF DIABETES AND ITS COMPLICATIONS, ELSEVIER SCIENCE, NEW YORK, NY, US, vol. 22, no. 2, 14 February 2008 (2008-02-14), pages 96 - 103, XP022479519, ISSN: 1056-8727, DOI: 10.1016/J.JDIACOMP.2007.08.001
- ZHANG JIN-BIAO ET AL: "Biomarkers of Renal Function in Type 2 Diabetic Patients with Cognitive Impairment", NEUROSCIENCE LETTERS, vol. 610, 28 October 2015 (2015-10-28), pages 19 - 23, XP029359990, ISSN: 0304-3940, DOI: 10.1016/J.NEULET.2015.10.059
- TONY HUSCHTO, HELENA K�NIG, CLAIRE E. MARRIOTT, DANIEL MILITZ, CHRISTIAN RINGEMANN: "Predicting the Risk of Chronic Kidney Disease in People with Diabetes (PWD) from Electronic Health Care Records (EHRs): A Validation Study", DIABETES, vol. 69, no. Supplement 1, 476-P, 1 June 2020 (2020-06-01), pages 476-P, XP009529690, DOI: 10.2337/db20-476-P
- AFSHAR NADER ET AL: "15-LB: Prediction of Chronic Kidney Disease (CKD) in People with Diabetes", DIABETES, vol. 70, no. Supplement 1, 21 June 2021 (2021-06-21) - 29 June 2021 (2021-06-29), US, pages 15 - LB, XP055835313, ISSN: 0012-1797, Retrieved from the Internet <URL:http://dx.doi.org/10.2337/db21-15-LB> DOI: 10.2337/db21-15-LB
- HOSSEINZADEH MEHDI ET AL: "A diagnostic prediction model for chronic kidney disease in internet of things platform", MULTIMEDIA TOOLS AND APPLICATIONS, KLUWER ACADEMIC PUBLISHERS, BOSTON, US, vol. 80, no. 11, 29 July 2020 (2020-07-29), pages 16933 - 16950, XP037458777, ISSN: 1380-7501, [retrieved on 20200729], DOI: 10.1007/S11042-020-09049-4

## Description

The present invention refers to a method for screening a subject for the risk of chronic kidney disease, a computer-implemented method, a system, and a computer program product.

### Background

In chronic kidney disease (CKD), kidney function is progressively lost, beginning with a decline in the glomerular filtration rate and / or albuminuria and progressing to end-stage renal disease. As a result, dialysis or renal transplant may be necessary (see Unger, J., Schwartz, Z., Diabetes Management in Primary Care, 2nd edition. Lippincott Williams & Wilkens, Philadelphia, USA, 2013). CKD is a serious problem, with an adjusted prevalence of 7% in 2013 (Glassock, R.J. et al., The global burden of chronic kidney disease: estimates, variability and pitfalls, Nat Rev Nephrol 13, 104-114, 2017). The early recognition of CKD could slow progression, prevent complications, and reduce cardiovascular-related outcomes (Platinga, L.C. et al., Awareness of chronic kidney disease among patients and providers, Adv Chronic Kidney Dis 17, 225-236, 2010). CKD may be a microvascular long-term complication of diabetes (Fioretto, P. et al., Residual microvascular risk in diabetes: unmet needs and future directions, Nat Rev Endocrinol 6, 19-25, 2010).

Algorithms for risk prediction of CKD by diabetic patients have been published, for example, by Dunkler et al. (Dunkler, D. et al., Risk Prediction for Early CKD in Type 2 Diabetes, Clin J Am Soc Nephrol 10, 1371-1379, 2015), Vergouwe et al. (Vergouwe, Y. et al., Progression to microalbuminuria in type 1 diabetes: development and validation of a prediction rule, Dia-betologia 53, 254-262, 2010), Keane et al. (Keane, W.F. et al., Risk Scores for Predicting Outcomes in Patients with Type 2 Diabetes and Nephropathy: The RENAAL Study, Clin J Am Soc Nephrol 1, 761-767, 2006) and Jardine et al (Jardine, M.J. et al., Prediction of Kidney-Related Outcomes in Patients With Type 2 Diabetes, Am J Kidney Dis. 60, 770-778, 2012). Such published algorithms are derived from data originating from major clinical studies.

Such predictive models based on clinical data represent an ideal setting with a preselected population, cross-checked and validated clinical data entries and often a narrow time window of observation. The outcomes therefore do not necessarily reveal the optimum pathways in terms of efficacy and effectiveness for a real-world population when inferred from clinical studies. In addition, most literature is focused on progression of diabetic nephropathy or CKD and therefore misses the early phase of this diabetic complication. Finally, patients are usually selected on the basis of a full set of respective features.

Document EP 3 543 702 A1 discloses a method for screening a subject for the risk of chronic kidney disease, comprises: receiving marker data indicative for a plurality of marker parameters for a subject, such plurality of marker parameters indicating, for the subject for a measurement period, an age value, a sample level of creatinine, and a sample level of albumin; and determining a risk factor indicative of the risk of suffering CKD for the subject from the plurality of marker parameters. The determining comprises weighting the age value higher than the sample level of albumin, and weighting the sample level of creatinine higher than the sample level of albumin. A computer-implemented method is disclosed applying logistic regression (LR) model for determining the risk of chronic kidney disease for a subject, such as a patient having received a diabetes diagnose. Document WO2019/180232 A1 discloses methods for screening a diabetes patient for the risk of chronic kidney disease, using the marker parameters creatinine, age, albumin, glomerular filtration rate, with a measurement period of two years and after diabetes diagnosis.

A method for longitudinal risk prediction of CKD in diabetic patients has been published by Song et al. (Song et al., Longitudinal risk prediction of chronic kidney disease in diabetic patients using a temporal-enhanced gradient boosting machine: retrospective cohort study, JMIR Med Inform 8(1), 2020, e15510). For predicting the risk of CKD a boosted machine learning model is proposed.

### Summary

It is an object of the present invention to provide an improved method for screening a subject for the risk of chronic kidney disease, allowing a reliable risk assessment for CKD based on real world data (RWD).

To solve this, a method for screening a subject for the risk of chronic kidney disease (CKD) according to the independent claim 1 is provided. Further, a computer-implemented method according to the independent claim 8 is provided. Also, a system and a computer program product according to claims 13 and 14, respectively, are provided. Additional embodiments are disclosed in the dependent claims.

According to an aspect, a method for screening a subject for the risk of chronic kidney disease (CKD) is provided, comprising receiving marker data indicative for a plurality of marker parameters for a subject, such plurality of marker parameters indicating at least the following: an age value, a time since diagnosis value indicative of a time since a diabetes diagnosis for the subject, a sample level of creatinine, an estimated glomerular filtration rate, a sample level of albumin, and a sample level of blood urea nitrogen. A risk factor indicative of the risk of suffering CKD is determined for the subject from the plurality of marker parameters.

According to another aspect, computer-implemented method for screening a subject for the risk of chronic kidney disease (CKD) in a data processing system is provided, the data processing system having a processor and a non-transitory memory storing a program causing the processor to execute: receiving marker data indicative for a plurality of marker parameters for a subject, such plurality of marker parameters indicating an age value, a time since diagnosis value indicative of a time since a diabetes diagnosis for the subject, a sample level of creatinine, a sample level of estimated glomerular filtration rate, a sample level of albumin, and a sample level of blood urea nitrogen; and determining a risk factor indicative of the risk of suffering CKD for the subject from the plurality of marker parameters.

A system is provided, comprising a processor and a non-transitory memory storing a program causing the processor to perform the method for screening a subject for the risk of chronic kidney disease (CKD).

A computer program or a computer program product is provided, comprising instructions which, when the program is executed by a computer, cause the computer to carry out steps of the method for screening a subject for the risk of chronic kidney disease (CKD).

The marker parameters may be indicative of real-world data which is not restricted regarding, for example, completeness or veracity of the data (unlike clinical data).

The time since diagnosis value refers to the time period from the time or date of an initial diabetes diagnosis for the subject to the date of determining the risk factor for the subject.

The method may further comprise the plurality of marker parameters indicating, for the subject, a blood sample level of creatinine. As the sample level of creatinine a serum sample level or a plasma sample may also be used. Thus, requesting the sample level of creatinine as a concentration in urine may be avoided. The plurality of marker parameters may indicate, for the subject, a selected blood sample level (or serum or plasma sample level) of creatinine selected from a plurality of blood sample levels (or serum or plasma sample levels, respectively) of creatinine. For example, the selected blood sample level of creatinine may be a maximum value from the plurality of blood sample levels of creatinine. Alternatively or additionally, the plurality of marker parameters may indicate, for the subject, a calculated blood sample level of creatinine calculated from a plurality of blood sample levels of creatinine. For example, the calculated blood sample level of creatinine may be a statistical value calculated from the plurality of blood sample levels of creatinine, such as a mean, median or mode value. The sample level of creatinine may be provided in units of mg / dl (such as milligrams of creatinine per deciliter of blood).

The method may further comprise the plurality of marker parameters indicating, for the subject, at least one of a blood sample level of albumin and a urine sample level of albumin. In one embodiment, the sample level of albumin is a blood sample level. As the sample level of albumin a serum sample level or a plasma sample may also be used. The plurality of marker parameters may also indicate, for the subject, a selected blood sample level (or serum or plasma sample level) of albumin selected from a plurality of blood sample levels (or serum or plasma sample levels, respectively) of albumin. For example, the selected blood sample level of albumin may be a minimum value from the plurality of blood sample levels of albumin. Alternatively or additionally, the plurality of marker parameters may indicate, for the subject, a calculated blood sample level of albumin calculated from a plurality of blood sample levels of albumin. For example, the calculated blood sample level of albumin may be a statistical value calculated from the plurality of blood sample levels of albumin, such as a mean, median or mode value. The sample level of albumin may be provided in units of mg / dl (such as milligrams of albumin per deciliter of blood).

The glomerular filtration rate is known in the art to be indicative of the flow rate of filtered fluid through the kidney. It is an important indicator for estimating renal function. The glomerular filtration rate may decrease due to renal disease. In embodiments, the glomerular filtration rate may be estimated using a Modification of Diet in Renal Disease (MDRD) formula, known in the art as such. For example, a MDRD formula using four variables relies on age, sex, ethnicity and serum creatinine of the subject for estimating glomerular filtration rate. In alternative embodiments, the glomerular filtration rate may be estimated using the CKD-EPI (Chronic Kidney Disease Epidemiology Collaboration) formula, known in the art as such. The CKD-EPI formula relies on age, sex, ethnicity and serum creatinine of the subject for estimating glomerular filtration rate. In further embodiments, the glomerular filtration rate may be estimated using other methods or may be directly determined. The estimated glomerular filtration rate may be provided in units of ml / min / 1.73m² (milliliters per minute per 1.73 square meters of body surface area).

The plurality of marker parameters may indicate, for the subject, a selected estimated glomerular filtration rate selected from a plurality of estimated glomerular filtration rates. For example, the selected estimated glomerular filtration rate may be a minimum value from the plurality of estimated glomerular filtration rates. Alternatively or additionally, the plurality of marker parameters may indicate, for the subject, a statistical value as the estimated glomerular filtration rated, calculated from a plurality of estimated glomerular filtration rates, such as a mean, median or mode value.

The sample level of blood urea nitrogen (BUN) may be provided in units of mg / dl (such as milligrams of urea nitrogen per deciliter of blood). The sample level of blood urea nitrogen (BUN) may thus represent the mass of nitrogen within urea/volume of the blood sample, not the mass of whole urea. The plurality of marker parameters may indicate, for the subject, a selected sample level of blood urea nitrogen, selected from a plurality of blood sample levels of urea nitrogen. For example, the selected blood sample level of urea nitrogen may be a minimum value from the plurality of blood sample levels of urea nitrogen. Alternatively or additionally, the plurality of marker parameters may indicate, for the subject, a calculated blood sample level of urea nitrogen calculated from a plurality of blood sample levels of urea nitrogen. For example, the calculated blood sample level of urea nitrogen may be a statistical value calculated from the plurality of blood sample levels of urea nitrogen, such as a mean, median or mode value.

The sample level of creatinine, the sample level of albumin, the sample level of blood urea nitrogen and / or the estimated glomerular filtration rate may be a representative sample level and / or rate from the respective plurality of sample levels and / or rates, such as a maximum sample level and / or rate, a minimum sample level and / or rate, a mean sample level and / or rate and / or a median of the sample levels and / or rates, respectively. In an exemplary embodiment, creatinine is a maximum sample level of creatinine from a plurality of sample levels of creatinine for the subject, albumin is a minimum sample level of albumin from a plurality of sample levels of albumin for the subject, eGFR is a minimum estimated glomerular filtration rate from a plurality of estimated glomerular filtration rates for the subject and blood urea nitrogen is a minimum sample level of blood urea nitrogen from a plurality of sample levels of blood urea nitrogen for the subject.

The marker data may stem from a measurement period of two years or less. The measurement period may thus be limited to two years. Thereby, values and / or sample levels of substances may be provided that have been collected within a time period of a maximum of two years with the risk factor indicating a risk of suffering CKD for the subject from the end of the measurement period onwards.

In one embodiment, at least the sample level of creatinine, the sample level of albumin, the sample level of blood urea nitrogen, and the estimated glomerular filtration rate stem from a measurement period of two years or less. The samples for determining the sample level of creatinine, the sample level of albumin, the sample level of blood urea nitrogen, and estimated glomerular filtration rate may have been taken and / or determined in a measurement period of two years or less.

The age value may correspond to the age of the patient (e.g., in years) when determining the risk factor.

The time since diagnosis value may be indicative of the time since a diabetes diagnosis for the subject when determining the risk factor. In one embodiment, the date of determining the risk factor for the subject may be defined as the end of the measurement period.

The risk factor indicative of the risk of suffering CKD for the subject is determined from the plurality of marker parameters, including at least the age value of the subject, the time since diagnosis value indicative of the time since the diabetes diagnosis for the subject, the sample level of creatinine of the subject, the estimated glomerular filtration rate of the subject, the sample level of albumin of the subject, and the sample level of blood urea nitrogen of the subject.

The risk factor may be indicative of the risk of suffering CKD for the subject within a prediction time period of three years from the end of the measurement period. The risk factor may be a probability for the subject of developing CKD within three years from the time the sample levels have been determined. Alternatively, the risk factor may be indicative of the risk of suffering CKD for the subject within a time period of less than three years, for example two years, from the end of the measurement period. As a further alternative, the risk factor may be indicative of the risk of suffering CKD for the subject within a time period of more than three years from the end of the measurement period.

With respect to the computer-implemented method, the determining of the risk factor may comprise the following: providing a machine learning model; providing input data indicative of the plurality of marker parameters to the machine learning model; and determining the risk factor by the machine learning model. Thus, the risk factor is determined by applying the machine learning model trained and tested (validated) before, such training / testing comprising training a machine learning algorithm for creating or determining the machine learning model being the result of such training including training and testing / validating.

The providing of the machine learning model may comprise providing an XGBoost machine learning model. XGBoost provides for a decision-tree-based ensemble machine learning algorithm that uses a gradient boosting framework. Gradient boosting is a machine learning technique for regression and classification problems, which produces a prediction model in the form of an ensemble of weak prediction models, typically decision trees. When a decision tree is the weak learner, the resulting algorithm is called gradient boosted trees, which usually outperforms random forest. It builds the model in a stage-wise fashion like other boosting methods do, and it generalizes them by allowing optimization of an arbitrary differentiable loss function.

The providing of the machine learning model may comprise, in a pre-processing, the following: providing a set of training data for a population of subjects, the training data being indicative of a plurality of training parameters for the population of subjects, wherein the training marker parameters comprising age, level of creatinine, level of estimated glomerular filtration rate, level of albumin, level of blood urea nitrogen, and an indicator whether the subject developed CKD; providing diabetes diagnosis data indicative of a time or date when a diabetes diagnosis was determined for subjects from the population of subjects; determining, from the diabetes diagnosis data, a supplementary training data indicating a time since diagnosis parameter indicative of a time since a diabetes diagnosis was determined for the subjects from the population of subjects; providing an augmented set of training data comprising the set of training data and the supplementary training data; and training the machine learning model such as the XGBoost machine learning model based on the augmented set of training data. The additional parameter referring to the time since diagnosis parameter is determined in a pre-processing, thereby, extending size and number of training data applied for training the machine learning model.

The pre-processing may also comprise a step of determining from the training data a set of preprocessed training data comprising one or more statistical values and / or selected values for one or more of the level of creatinine, estimated glomerular filtration rate, level of albumin, and level of blood urea nitrogen for a respective one or more subjects from the population of subjects. For example, for a subject of the population a plurality of sample levels of creatinine may have been determined. In the preprocessing, one or more statistical values and / or selected values may thus be determined from the plurality of sample levels of creatinine, such as a mean creatinine value and / or a maximum creatinine value for that subject from the population.

A method of training a machine learning model for the determination of a risk factor indicative of the risk of suffering CKD for a test subject from a plurality of marker parameters of the test subject is also provided herein. The method of training comprises:
- providing a set of training data for a population of training subjects, the training data being indicative of a plurality of training parameters for the population of training subjects, wherein the training marker parameters comprising at least: age, level of creatinine, level of estimated glomerular filtration rate, level of albumin, and level of blood urea nitrogen, and the training data further comprising for each training subject an indication whether the training subject developed CKD;
- optionally determining from the training data a set of preprocessed training data comprising one or more one or more statistical values and / or selected values for one or more of the level of creatinine, estimated glomerular filtration rate, level of albumin, and level of blood urea nitrogen for respective training subjects from the population of training subjects;
- providing diabetes diagnosis data indicative of a time or date when a diabetes diagnosis was determined for respective training subjects from the population of training subjects;
- determining, from the diabetes diagnosis data, supplementary training data indicating a time since diagnosis parameter indicative of a time since a diabetes diagnosis was determined for the respective training subjects from the population of training subjects;
- providing an augmented set of training data comprising
   - the set of training data and / or the set of preprocessed training data and
   - the supplementary training data; and
- training the machine learning model based on the augmented set of training data for the determination of the risk factor indicative of the risk of suffering CKD for a test subject.

A method for screening a test subject for the risk of chronic kidney disease (CKD) is further provided herein, comprising
- training a machine learning model according to the method of training as described above and thereby obtaining a trained machine learning model;
- receiving marker data indicative for a plurality of marker parameters for the test subject, such plurality of marker parameters indicating at least the following: an age value, a time since diagnosis value indicative of a time since a diabetes diagnosis for the subject, a sample level of creatinine, an estimated glomerular filtration rate, a sample level of albumin, and a sample level of blood urea nitrogen; and
- determining a risk factor indicative of the risk of suffering CKD for the test subject from the plurality of marker parameters by using the trained machine learning model.

The risk factor may be determined using a machine learning model, wherein no marker data are imputed. Thus, machine learning model was trained / tested by training (and testing or validating) data free of imputed marker data.

Within the meaning of the present disclosure, screening a subject for the risk of CKD means identifying a subject at risk of developing or having CKD.

A sample level in the sense of the present disclosure is a level of a substance, such as creatinine or albumin, in a sample of a bodily fluid of the subject. Sample levels may be determined in the same or different samples. Alternatively or additionally, for determining sample levels, measurements may be performed in the same or different samples. For example, a sample level of a substance may be determined from a plurality of measurements of the same substance in the same sample, for example by determining a mean value. In another example, at least one of a plurality of sample levels of the same substance may be determined in a first sample and at least another one of the plurality of sample levels of the same substance may be determined in a second sample. A sample level of a first substance and a sample level of a second substance may be determined in the same sample. Alternatively, a sample level of a first substance may be determined in a first sample and a sample level of a second substance may be determined in a second sample.

A computer program product may be provided, including a computer readable medium embodying program code executable by a process of a computing device or system, the program code, when executed, causing the computing device or system to perform the computer-implemented method for screening a subject for the risk of chronic kidney disease.

With regard to the computer-implemented method, the alternative embodiments described above may apply *mutatis mutandis.*

In the computer-implemented method, the program may further cause the processor to execute generating output data indicative of the risk factor and outputting the output data to an output device of the data processing system. The output device may be any device suitable for outputting the output data, for example a display device of the data processing system, such as a monitor, and / or a transmitter device for transmitting for wired and / or wireless data transmission. The output data may be output to a user, for example a physician, via a display of the data processing system. Based on the output data indicative of the risk factor, further marker data may be requested from the subject and / or a future date for a further screening of the subject for CKD may be set (e.g., then based on at least one or more newly collected sample levels of one or more of creatinine, albumin, blood urea nitrogen and / or a newly determined estimated glomerular filtration rate, new age value, new time since diagnosis value indicative of the time since the diabetes diagnosis for the subject considering the future date).

The data processing system may comprise a plurality of data processing devices, each data processing device having a processor and a memory. The marker data may be provided in a first data processing device. For example, the marker data may be received in the first data processing device by user input via an input device and / or by data transfer. The marker data may be sent from the first data processing device to a second data processing device which may be located remotely with respect to the first data processing device. The marker data may be received in the second data processing device and the risk factor may then be determined in the second data processing device. Result data indicative of the risk factor may be sent from the second data processing device to the first data processing device or, alternatively or additionally, to a third data processing device. The result data may then be stored in the first and / or the third data processing device and / or output via an output device of the first and / or the third data processing device.

The first data processing device and / or the third data processing device may be a local device, such as a client computer, and the second data processing device may be a remote device, such as a remote server.

Alternatively, the functionality of at least the first data processing device and the second data processing device may be provided in the same data processing device, for example a computer, such as a computer in a physician's office. All steps of the computer-implemented method may be executed in the same data-processing device.

### Description of further embodiments

Following, embodiments are described by way of example. Reference is made to figures. In the figures show:
- Fig. 1: a schematic representation for determining an XGBoost machine learning model;
- Fig. 2: a schematic representation for a computer-implemented method for determining a risk factor indicative of a risk of CKD for a subject; and
- Fig. 3: ROC curve for the "Full XG boost model", the "Top 20 XG boost model", and the "LR Top 20 model" for both using all parameters and using only limited number of parameters.

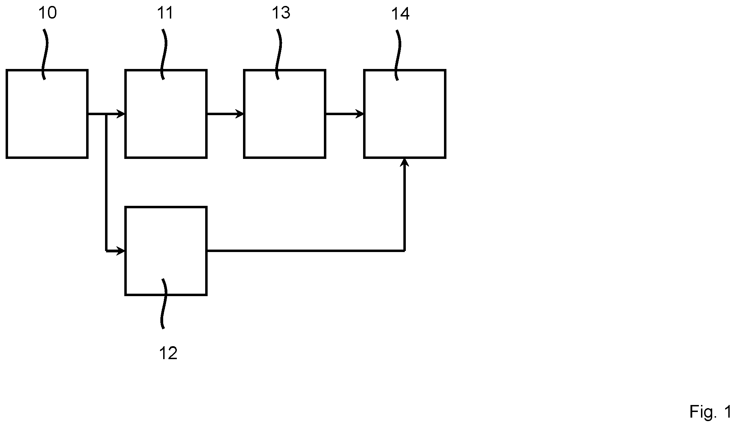

Fig. 1 shows a schematic representation for determining or creating - by training and testing / validating a machine learning algorithm - a machine learning model, the machine learning model implemented, in an example, with a an XGBoost machine learning model. A data set for a population of subjects is provided in step 10.

A machine learning model is created using electronic health record (EHR) data, for example from several hundred thousands of people with diabetes (type 1 or type 2) represented in a database. The data is retrieved for the time window after the initial diagnosis of diabetes. The data can be considered as real-world data (RWD) and no general restrictions on, for example, completeness or veracity of the data are applied.

No missing data are imputed for teaching or learning (training and testing / validating) the model. An XGBoost machine learning process has been applied.

In an example the data set was provided from the so-called IBM Explorys database (see Kaelber, D.C. et al., Patient characteristics associated with venous thromboembolic events: a cohort study using pooled electronic health record data, J Am Med Inform Assoc 19, 965-972, 2012). An alternative example for a data set for a population of subjects is the Indiana Network for Patient Care (INPC) database (see McDonald, C. J. et al., The Indiana Network for Patient Care: a working local health information infrastructure, Health Affairs 24, 1214-1220, 2005).

The database provides indication as to a date of diabetes diagnosis for the subjects. Starting from such information a new parameter is established such parameter providing indication of a time period since diabetes diagnosis for the respective subject. A pre-processing step is applied for determining such additional parameter from the diabetes diagnosis data provided in the database. It provides for a supplementary training data indicating a time since diagnosis parameter indicative of the time since a diabetes diagnosis was determined for the subjects from the population of subjects. Thus, there is an augmented set of training data comprising in addition the supplementary training data.

From the data set including supplementary training data indicating the time since diagnosis parameter, a set of training data and a set of test / validation data are determined (steps 11, 12). The set of training data is indicative of a plurality of parameters for the population of subjects (step 11). With regard to the data set provided for the population of subjects, the set of training data may comprise training data indicative of (almost) all parameters for which data are provided in the data set of the population of subjects. Alternatively, a subset of parameters may be selected for training of the machine learning model.

Following, there is a training process for a machine learning model in step 13 based on the set of training data. In an example, in the training process a XBoost training is applied for determining or creating a XBoost machine learning model. The machine learning model is finally determined in step 14 applying the set of test / validation data for final model evaluation.

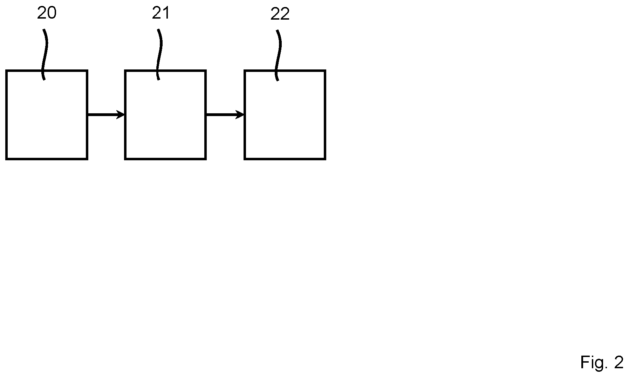

Fig. 2 shows a schematic representation with respect to a computer-implemented method for determining a risk factor indicative of a risk of chronic kidney decease (CKD) for a subject. In step 20 marker data are provided which are indicative of a plurality of marker parameters for the subject for which the risk factor is to be determined. In an example, the plurality of marker parameters is indicative of: an age value, a time since diagnoses value indicative of a time since a diabetes diagnoses for the subject, a sample level of creatinine, an estimated glomerular filtration rate (eGFR), a sample level of albumin, and a sample level of blood urea nitrogen (BUN). The marker data are provided as an input to the machine learning model (step 21). By applying the machine learning model a risk factor for the risk of chronic kidney decease for the subject is determined (step 22). The machine learning model is implemented by a software application on a data processing device having a processor and a memory.

In general, in any of the embodiments of the method for screening a subject for the risk of CKD, creatinineₘₐₓ may be a maximum sample level of creatinine from a plurality of sample levels of creatinine for the subject, albuminₘᵢₙ may be a minimum sample level of albumin from a plurality of sample levels of albumin for the subject, eGFRₘᵢₙ may be a minimum estimated glomerular filtration rate from a plurality of estimated glomerular filtration rates for the subject, BUNₘᵢₙ may be a minimum blood sample level of urea nitrogen. Such values and / or sample levels may be determined from values and / or sample levels already on file for the subject. Alternatively or in addition, values and / or sample levels may be determined for the subject specifically for use with the method for screening a subject for the risk of CKD. Values and / or sample levels may be real world data, i.e., unlike clinical data, they may not be restricted regarding, for example, completeness or veracity of the data.

ICD codes may be used as target variables for training as well as the CKD reference diagnosis in the analysis of the validation results. The definition of the target feature "CKD" may be solely based on the occurrence of the respective ICD codes in the databases. In order to maintain the RWD character of the data set, no additions or changes may be made to the databases. Such ICD codes may comprise ICD-9 codes and ICD-10 codes, for example the following ICD codes: 250.40, 250.41, 250.42, 250.43, 585.1, 585.2, 585.3, 585.4, 585.5, 585.6, 585.9, 403.00, 403.01, 403.11, 403.90, 403.91, 404.0, 404.00, 404.01, 404.02, 404.03, 404.1, 404.10, 404.11, 404.12, 404.13, 404.9, 404.90, 404.91, 404.92, 404.93, 581.81, 581.9, 583.89, 588.9, E10.2, E10.21, E10.22, E10.29, E11.2, E11.21, E11.22, E11.29, N17.0, N17.1, N17.2, N17.8, N17.9, N18.1, N18.2, N18.3, N18.4, N18.5, N18.6, N18.9, N19, 112.0, 112.9, 113, 113.0, 113.1, 113.10, 113.11, 113.2, N04.9, N05.8, N08 and / or N25.9.

In an embodiment, the ICD-9 codes 250.40, 403.90, 585.3, 585.9 are the most abundant diagnosis in the respective time windows of the data.

ICD codes may also be used to determine a diabetes diagnosis. E.g., type 1 diabetes diagnosis may be based on ICD-9 codes 250._1 and/or 250._3, and/or ICD-10 codes E10.%. E.g., type 2 diabetes diagnosis may be based on ICD-9 codes 250._0 and/or 250._2, and/or ICD-10 codes E11.%. "_" and "%" are placeholders, wherein "_" may not be empty; However, the placeholder "%" may be empty.

### Experimental data

The area under the receiver operating characteristic (ROC) (compare Swets, J.A., Measuring the accuracy of diagnostic systems, Science 240,1285-1293, 1988) curve (AUC) is frequently used to measure the quality of clinical markers as well as machine learning algorithms / models (see Bradley, A.P., The use of the area under the ROC curve in the evaluation of machine learning algorithms, Pattern Recognition 30, 1145-1159, 1997). A perfect marker would achieve AUC=1.0, whereas flipping a coin would result in AUC=0.5.

Machine learning models applying XGBoost in the learning procedure have been trained and tested based on different sets of training data referring to all parameters available in the database or a subset of the parameters. A machine learning model referred to as "Full XG boost model" has been trained using all parameters from the plurality of parameters such as about 100 (about 948 features) available in the IBM Explorys database. Parameters in this context refers to, e.g., creatinine, albumin, age etc. Features in this context, e.g., refers to selected or statistical values, such creatinineₘₐₓ or creatinine_{median}. The "Full XG boost model" was created by using all available parameters (all features). With respect to the data from the database, not all parameters are available for every patient (subject) of the population. When working with the full set of parameters it was found that certain parameters are particularly important (e.g. top 5 or top 20 or top 30).

Further, a machine learning model referred to as "Top 20 XG boost model" has been created (training and testing) using only a subset of the data from the IBM Explorys database. In an embodiment, the data of the subset of data are relating to (only) 20 parameters from the plurality of parameters, the 20 parameters being parameters which were found most important in the machine learning process for the "Full XG boost model". Following, such 20 parameters are listed (not in an order of importance): age; albumin (serum and / or plasma); albumin (urine), systolic blood pressure, blood urea nitrogen (BUN), medication with antihypertensive drugs, medication with insulin; number of pre-existing conditions of: diabetic retinopathy, ischemic heart disease, peripheral artery occlusive disease, cerebrovascular disease; creatinine (serum / plasma); time (days) since diabetes diagnosis; mean time span between two doctor's visits where a parameter has been measured or a diagnosis has been made; diagnosis with DM type 2 with hyperglycemia; diagnosis of heart failure; estimated glomerular filtration rate (eGFR); erythrocytes (serum and / or plasma); glucose (serum and / or plasma); hematocrit; hemoglobin; urine albumin-to-creatinine ratio (UACR); and body weight.

In the training (learning procedure) of the "Top 20 XG boost model" created as a separate model, only the top 20 parameters determined from the training of the "Full XGBoost model" were used. Thus, other parameters (even though possibly available) were ignored when the "Top 20 XG boost model" was determined.

For evaluating the machine learning models for both the "Full XG boost model" and the "Top 20 XG boost model" AUC was determined for population of subjects for which the database provides real world data. Such calculation was performed for the population of subjects taking into account all parameters (features) available. In addition, the calculation was performed for the population of subjects taking into account only data related to the following (six) parameters: age, time since diabetes, creatinine, estimated glomerular filtration rate (eGFR), albumin, and blood urea nitrogen (BUN) ("using limited number of parameters").

For comparison, AUC was calculated for a logistic regression (LR) model also trained based on the data of the subset of data relating to the 20 parameters from the plurality of parameters. Such machine learning model is referred to as "LR Top 20 model". For the "LR Top 20 model (only limited number of parameters)", the AUC calculation and specificity @ 90% Sensitivity were assessed by considering only the subject specific data relating to the following (six) parameters: age, time since diabetes, creatinine, estimated glomerular filtration rate (eGFR), albumin, and blood urea nitrogen (BUN) ("using limited number of parameters"). For the 14 other parameters, no subject specific data were used, but for those other parameters data were imputed from cohorts selected or statistical values, respectively.

The performance of a method for screening a subject for the risk of CKD or for identifying those people at high risk of developing CKD may be judged according to sensitivity (fraction of correctly predicted high-risk patients) and specificity (fraction of correctly assigned low-risk patients). However, either of these numbers can be improved at the expense of the other simply by changing the threshold between high and low risk. Hence, data pairs of sensitivity and specificity may be illustrated in forms of the so-called receiver operating characteristic (ROC) curve (see Swets, J.A., Measuring the accuracy of diagnostic systems, Science 240, 1285-1293, 1988) in which the sensitivity is plotted as a function of 1-specificity (which corresponds to the fraction of falsely assigned high-risk persons).

Results of the calculations conducted for all parameters or only the six parameters identified above for the data from the Indiana Network for Patient Care (INPC) database are shown in Table 1.

**Table 1**

| | AUC | Specificity @ 90% Sensitivity |
|---|---|---|
| "Full XGBoost model" (using all features) | 0.849 | 0.555 |
| "Top20 XGBoost model" (using all features) | 0.842 | 0.537 |
| "Full XGBoost Model" (using only limited number of parameters) | 0.828 | 0.499 |
| "Top20 XGBoost Model" (using only limited number of parameters) | 0.823 | 0.484 |
| "LR Top 20 model" (using all features) | 0.819 | 0.470 |
| "LR Top 20 Model" (only limited number of parameters) | 0.809 | 0.441 |

As can be seen from Table 1, AUC for the machine learning models are high for all depicted models, but by applying XGBoost even better results could be achieved than for the LR model. Using only the limited number of parameters for calculating AUC still provides reliable result.

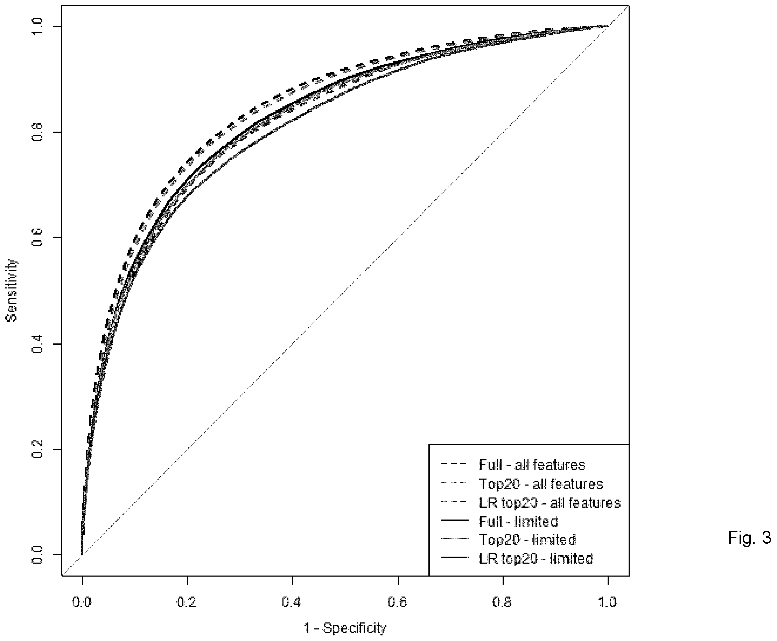

Fig. 3 shows the ROC curve for the "Full XG boost model", the "Top 20 XG boost model", and the "LR Top 20 model" for both using all parameters and using only limited number of parameters. For a perfect classifier, the ROC curve reaches the upper-left corner. In fact, the threshold corresponding to the data pair closest to this corner is dubbed the "optimal threshold". When aiming for high sensitivity, an alternative threshold may be chosen to guarantee a sensitivity of, for example, 90%.

For additionally comparing the machine leaned XGBoost model presented here, calculations were also conducted for a model (algorithm) for predicting a risk factor for CKD known from EP 3 543 702 A1, the model referred to as "Algorithm model" in the following. The "Algorithm model" also applies logistic regression. No data imputation was applied. Results of the calculations conducted for data from the IBM Explorys database are shown in Table 2.

**Table 2**

| | AUC | Specificity @ 90% Sensitivity |
|---|---|---|
| "Full XGBoost model" (using all features) | 0.836 | 0.519 |
| "Top20 XGBoost model" (using all features) | 0.829 | 0.499 |
| "Algorithm model" | 0.769 | 0.347 |

From Table 2 it is concluded that the machine learning model applying XGBoost provides improved results in terms of risk factor determination over the "Algorithm model".

In summary, it is demonstrated that different machine learning models for predicting a risk factor for CKD performed robust even if only a limited number of marker parameters is available (specific selection of marker parameters): age, time since diabetes, creatinine, estimated glomerular filtration rate (eGFR), albumin, and blood urea nitrogen (BUN). The results support the path towards high-quality predictive models that can be applied in a clinical setting, enabling the shift towards personalized and outcome-based healthcare.

## Claims

1. A method for screening a subject for the risk of chronic kidney disease (CKD), comprising
- receiving marker data indicative for a plurality of marker parameters for a subject, such plurality of marker parameters indicating at least
- an age value,
- a time since diagnosis value indicative of a time since a diabetes diagnosis for the subject,
- a sample level of creatinine,
- an estimated glomerular filtration rate,
- a sample level of albumin, and
- a sample level of blood urea nitrogen; and
- determining a risk factor indicative of the risk of suffering CKD for the subject from the plurality of marker parameters.

2. The method of claim 1, further comprising the plurality of marker parameters indicating, for the subject, a blood sample level of creatinine.

3. The method of claim 1 or 2, further comprising the plurality of marker parameters indicating, for the subject, at least one of a blood sample level of albumin and an urine sample level of albumin.

4. The method of any one of the preceding claims, wherein the receiving comprises receiving marker data indicative for a plurality of marker parameters for the subject for a measurement period of two years or less.

5. The method of any one of the preceding claims, wherein the age value corresponds to the age of the subject when determining the risk factor.

6. The method of any one of the preceding claims, wherein the time since diagnosis value is indicative of the time since the diabetes diagnosis for the subject when determining the risk factor.

7. The method of any one of the preceding claims, wherein the risk factor is indicative of the risk of suffering CKD for the subject within a prediction time period of three years from the end of the measurement period.

8. A computer-implemented method for screening a subject for the risk of chronic kidney disease (CKD) in a data processing system having a processor and a non-transitory memory storing a program causing the processor to execute:
a) receiving marker data indicative for a plurality of marker parameters for a subject, such plurality of marker parameters indicating at least
- an age value,
- an value indicating a time since a diabetes diagnosis for the subject,
- a sample level of creatinine,
- an estimated glomerular filtration rate,
- a sample level of albumin, and
- a sample level of blood urea nitrogen; and
b) determining a risk factor indicative of the risk of suffering CKD for the subject from the plurality of marker parameters.

9. The computer-implemented method of claim 8, wherein the determining of the risk factor in step b) comprises
- providing a machine learning model;
- providing input data indicative of the plurality of marker parameters to the machine learning model; and
- determining the risk factor by the machine learning model.

10. The computer-implemented method of claim 9, wherein the machine learning model comprises providing an XGBoost machine learning model.

11. The computer-implemented method of any of claims 8 to 10, wherein the providing of the machine learning model comprises
- providing a set of training data for a population of subjects, the training data being indicative of a plurality of training parameters for the population of subjects, wherein the training parameters are comprising: age, level of creatinine, estimated glomerular filtration rate, level of albumin, level of blood urea nitrogen, and an indicator whether the subject developed CKD;
- providing diabetes diagnosis data indicative of a time or date when a diabetes diagnosis was determined for subjects from the population of subjects;
- determining, from the diabetes diagnosis data, a supplementary training data indicating a time since diagnosis parameter indicative of a time since a diabetes diagnosis was determined for the subjects from the population of subjects;
- providing an augmented set of training data comprising the set of training data and the supplementary training data; and
- training the machine learning model based on the augmented set of training data.

12. The computer-implemented method of any of claims 8 to 11, wherein the risk factor is determined using the machine learning model with no marker data imputed.

13. A system comprising a processor and a non-transitory memory storing a program causing the processor to perform steps a) and b) of the method of any one of claims 8 to 12 for screening a subject for the risk of chronic kidney disease (CKD).

14. A computer program or a computer program product comprising instructions which, when the program is executed by a computer, cause the computer to carry out steps a) and b) of the method of any one of claims 8 to 12 for screening a subject for the risk of chronic kidney disease (CKD).

## Patentansprüche

1. Verfahren zum Screenen einer Person auf das Risiko einer chronischen Nierenerkrankung (CKD), umfassend
- Empfangen von Markerdaten, die eine Mehrzahl von Markerparametern für eine Person anzeigen, wobei eine solche Mehrzahl von Markerparametern mindestens Folgendes angibt:
- einen Alterswert,
- einen Zeit-seit-Diagnose-Wert, der eine Zeit seit einer Diabetes-Diagnose für die Person angibt,
- einen Probenspiegel von Kreatinin,
- eine geschätzte glomeruläre Filtrationsrate,
- einen Probenspiegel von Albumin und
- einen Probenspiegel von Blut-Harnstoff-Stickstoff; und
- Bestimmen eines Risikofaktors, der das Risiko für die Person angibt, CKD zu erleiden, aus der Mehrzahl von Markerparametern.

2. Verfahren nach Anspruch 1, ferner umfassend die Mehrzahl von Markerparametern, die für die Person einen Blutprobenspiegel von Kreatinin anzeigen.

3. Verfahren nach Anspruch 1 oder 2, ferner umfassend die Mehrzahl von Markerparametern, die für die Person mindestens einen von einem Blutprobenspiegel von Albumin und einem Urinprobenspiegel von Albumin anzeigen.

4. Verfahren nach einem der vorhergehenden Ansprüche, wobei das Empfangen das Empfangen von Markerdaten umfasst, die eine Mehrzahl von Markerparametern für die Person für einen Messzeitraum von zwei Jahren oder weniger anzeigen.

5. Verfahren nach einem der vorhergehenden Ansprüche, wobei der Alterswert dem Alter der Person entspricht, wenn der Risikofaktor bestimmt wird.

6. Verfahren nach einem der vorhergehenden Ansprüche, wobei der Zeit-seit-Diagnose-Wert die Zeit seit der Diabetes-Diagnose für die Person anzeigt, wenn der Risikofaktor bestimmt wird.

7. Verfahren nach einem der vorhergehenden Ansprüche, wobei der Risikofaktor das Risiko für die Person anzeigt, innerhalb eines Vorhersagezeitraums von drei Jahren ab dem Ende des Messzeitraums CKD zu erleiden.

8. Computerimplementiertes Verfahren zum Screenen einer Person auf das Risiko einer chronischen Nierenerkrankung (CKD) in einem Datenverarbeitungssystem mit einem Prozessor und einem nichtflüchtigen Speicher, der ein Programm speichert, das den Prozessor veranlasst, Folgendes auszuführen:
a) Empfangen von Markerdaten, die eine Mehrzahl von Markerparametern für eine Person angeben, wobei eine solche Mehrzahl von Markerparametern mindestens Folgendes angibt:
- einen Alterswert,
- einen Wert, der eine Zeit seit einer Diabetes-Diagnose für die Person anzeigt,
- einen Probenspiegel von Kreatinin,
- eine geschätzte glomeruläre Filtrationsrate,
- einen Probenspiegel von Albumin und
- einen Probenspiegel von Blut-Harnstoff-Stickstoff; und
b) Bestimmen eines Risikofaktors, der das Risiko für die Person angibt, CKD zu erleiden, aus der Mehrzahl von Markerparametern.

9. Computerimplementiertes Verfahren nach Anspruch 8, wobei das Bestimmen des Risikofaktors in Schritt b) Folgendes umfasst:
- Bereitstellen eines Modells für maschinelles Lernen;
- Bereitstellen von Eingangsdaten, die die Mehrzahl von Markerparametern für das Modell für maschinelles Lernen angeben; und
- Bestimmen des Risikofaktors mittels des Modells für maschinelles Lernen.

10. Computerimplementiertes Verfahren nach Anspruch 9, wobei das Modell für maschinelles Lernen das Bereitstellen eines XGBoost-Modells für maschinelles Lernen umfasst.

11. Computerimplementiertes Verfahren nach einem der Ansprüche 8 bis 10, wobei das Bereitstellen des Modells für maschinelles Lernen Folgendes umfasst:
- Bereitstellen eines Satzes von Trainingsdaten für eine Population von Personen, wobei die Trainingsdaten eine Mehrzahl von Trainingsparametern für die Population von Personen angeben, wobei die Trainingsparameter Folgendes umfassen: Alter, Kreatininspiegel, geschätzte glomeruläre Filtrationsrate, Albuminspiegel, Blut-Harnstoff-Stickstoffspiegel und einen Indikator, ob die Person CKD entwickelt hat;
- Bereitstellen von Daten zur Diabetesdiagnose, die den Zeitpunkt oder das Datum anzeigen, zu dem bei Personen aus der Population von Personen eine Diabetesdiagnose gestellt wurde;
- Bestimmen von zusätzlichen Trainingsdaten, die einen Zeit-seit-DiagnoseParameter angeben, der eine Zeit seit einer Diabetesdiagnose für die Personen aus der Population von Personen angibt;
- Bereitstellen eines erweiterten Satzes von Trainingsdaten, der den Satz von Trainingsdaten und die zusätzlichen Trainingsdaten umfasst; und
- Trainieren des Modells für maschinelles Lernen auf der Grundlage des erweiterten Satzes von Trainingsdaten.

12. Computerimplementiertes Verfahren nach einem der Ansprüche 8 bis 11, wobei der Risikofaktor unter Verwendung des Modells für maschinelles Lernen ohne zugerechnete Markerdaten bestimmt wird.

13. System, umfassend einen Prozessor und einen nichtflüchtigen Speicher, der ein Programm speichert, das den Prozessor veranlasst, die Schritte a) und b) des Verfahrens nach einem der Ansprüche 8 bis 12 zum Screenen einer Person auf das Risiko einer chronischen Nierenerkrankung (CKD) durchzuführen.

14. Computerprogramm oder Computerprogrammprodukt, umfassend Anweisungen, die den Computer veranlassen, die Schritte a) und b) des Verfahrens nach einem der Ansprüche 8 bis 12 zum Screenen einer Person auf das Risiko einer chronischen Nierenerkrankung (CKD) durchzuführen, wenn das Programm von einem Computer ausgeführt wird.

## Revendications

1. Procédé de criblage d'un sujet pour évaluer le risque d'une maladie rénale chronique (MRC), comprenant
- la réception de données de marqueur indicatives d'une pluralité de paramètres de marqueur pour un sujet, une telle pluralité de paramètres de marqueur indiquant au moins
- une valeur d'âge,
- une valeur de temps depuis le diagnostic indicative d'un temps depuis un diagnostic de diabète pour le sujet,
- un taux de créatinine dans un échantillon,
- un débit de filtration glomérulaire estimé,
- un taux d'albumine dans un échantillon, et
- un taux d'azote urique sanguin dans un échantillon ; et
- la détermination d'un facteur de risque indicatif du risque de souffrir de MRC pour le sujet à partir de la pluralité de paramètres de marqueur.

2. Procédé selon la revendication 1, comprenant en outre la pluralité de paramètres de marqueur indiquant, pour le sujet, un taux de créatinine dans un échantillon sanguin.

3. Procédé selon la revendication 1 ou 2, comprenant en outre la pluralité de paramètres de marqueur indiquant, pour le sujet, au moins un parmi un taux d'albumine dans un échantillon sanguin et un taux d'albumine dans un échantillon urinaire.

4. Procédé selon l'une quelconque des revendications précédentes, dans lequel la réception comprend la réception de données de marqueur indicatives d'une pluralité de paramètres de marqueur pour le sujet pour une période de mesure de deux ans ou moins.

5. Procédé selon l'une quelconque des revendications précédentes, dans lequel la valeur d'âge correspond à l'âge du sujet lors de la détermination du facteur de risque.

6. Procédé selon l'une quelconque des revendications précédentes, dans lequel la valeur de temps depuis le diagnostic est indicative du temps depuis le diagnostic de diabète pour le sujet lors de la détermination du facteur de risque.

7. Procédé selon l'une quelconque des revendications précédentes, dans lequel le facteur de risque est indicatif du risque de souffrir de MRC pour le sujet dans une période de temps de prévision de trois ans à partir de la fin de la période de mesure.

8. Procédé mis en œuvre par ordinateur de criblage d'un sujet pour évaluer le risque de maladie rénale chronique (MRC) dans un système de traitement de données ayant un processeur et une mémoire non transitoire stockant un programme amenant le processeur à exécuter:
a) la réception de données de marqueur indicatives d'une pluralité de paramètres de marqueur pour un sujet, une telle pluralité de paramètres de marqueur indiquant au moins
- une valeur d'âge,
- une valeur indicative d'un temps depuis un diagnostic de diabète pour le sujet,
- un taux de créatinine dans un échantillon,
- un débit de filtration glomérulaire estimé,
- un taux d'albumine dans un échantillon, et
- un taux d'azote urique sanguin dans un échantillon ; et
b) la détermination d'un facteur de risque indicatif du risque de souffrir de MRC pour le sujet à partir de la pluralité de paramètres de marqueur.

9. Procédé mis en œuvre par ordinateur selon la revendication 8, dans lequel la détermination du facteur de risque à l'étape b) comprend
- la fourniture d'un modèle d'apprentissage automatique ;
- la fourniture de données d'entrée indicatives de la pluralité de paramètres de marqueur au modèle d'apprentissage automatique ; et
- la détermination du facteur de risque par le modèle d'apprentissage automatique.

10. Procédé mis en œuvre par ordinateur selon la revendication 9, dans lequel le modèle d'apprentissage automatique comprend la fourniture d'un modèle d'apprentissage automatique XGBoost.

11. Procédé mis en œuvre par ordinateur selon l'une quelconque des revendications 8 à 10, dans lequel la fourniture du modèle d'apprentissage automatique comprend
- la fourniture d'un ensemble de données d'entraînement pour une population de sujets, les données d'entraînement étant indicatives d'une pluralité de paramètres d'entraînement pour la population de sujets, dans lequel les paramètres d'entraînement comprennent : l'âge, le taux de créatinine, le débit de filtration glomérulaire estimé, le taux d'albumine, le taux d'azote urique sanguin, et un indicateur indiquant si le sujet a développé une MCR ;
- la fourniture de données de diagnostic de diabète indicatives d'une heure ou d'une date à laquelle un diagnostic de diabète a été déterminé pour les sujets issus de la population de sujets ;
- la détermination, à partir des données de diagnostic de diabète, de données d'entraînement supplémentaires indiquant un paramètre de temps depuis le diagnostic indicatif d'un temps depuis la détermination d'un diagnostic de diabète pour les sujets issus de la population de sujets ;
- la fourniture d'un ensemble augmenté de données d'entraînement comprenant l'ensemble de données d'entraînement et les données d'entraînement supplémentaires ; et
- l'entraînement du modèle d'apprentissage automatique en se basant sur l'ensemble augmenté de données d'entraînement.

12. Procédé mis en œuvre par ordinateur selon l'une quelconque des revendications 8 à 11, dans lequel le facteur de risque est déterminé en utilisant le modèle d'apprentissage automatique sans aucune donnée de marqueur imputée.

13. Système comprenant un processeur et une mémoire non transitoire stockant un programme amenant le processeur à réaliser les étapes a) et b) du procédé selon l'une quelconque des revendications 8 à 12 de criblage d'un sujet pour évaluer le risque de maladie rénale chronique (MRC).

14. Programme informatique ou produit de programme informatique comprenant des instructions qui, lorsque le programme est exécuté par un ordinateur, amènent l'ordinateur à réaliser les étapes a) et b) du procédé selon l'une quelconque des revendications 8 à 12 de criblage d'un sujet pour évaluer le risque de maladie rénale chronique (MRC).
